# EUROPEAN PATENT SPECIFICATION

(11) **EP 4 035 180 B1**
(45) Date of publication and mention of the grant of the patent: **08.11.2023**
(21) Application number: 20771583.0
(22) Date of filing: 18.09.2020
(51) Int. Cl.: G16H 50/30, A61B 5/00, G08B 21/02, G16H 50/70

(54) **A COMPUTER-IMPLEMENTED SYSTEM AND METHOD FOR ALERTING AN EXPECTANT MOTHER TO A MEDICAL RISK DURING PREGNANCY**
COMPUTERIMPLEMENTIERTES SYSTEM UND VERFAHREN ZUR WARNUNG EINER WERDENDEN MUTTER BEI EINEM MEDIZINISCHEN RISIKO WÄHREND DER SCHWANGERSCHAFT
SYSTÈME INFORMATIQUE ET PROCÉDÉ PERMETTANT D'ALERTER UNE FUTURE MAMAN SUR UN RISQUE MÉDICAL PENDANT LA GROSSESSE

(30) Priority: 26.09.2019 EP 19199766
(43) Date of publication of application: 03.08.2022
(73) Proprietor: Koninklijke Philips N.V., 5656 AG Eindhoven (NL)
(72) Inventor: LUI, Wing, Lam, 5656 AE Eindhoven (NL); BRAND, Reon, 5656 AE Eindhoven (NL); VAN DER WOUDE, Daisy, Adrian, Anne, Jan, 5656 AE Eindhoven (NL); OEI, Swun, Gie, 5656 AE Eindhoven (NL)
(74) Representative: Philips Intellectual Property & Standards
(86) International application number: PCT/EP2020/076091
(87) International publication number: WO 2021/058389

(56) References cited:
- WO-A1-2018/071845
- DE-A1- 19 955 208
- DE-B4- 19 955 208

## Description

### FIELD OF THE INVENTION

This invention relates to health monitoring systems, and particularly for expectant mothers.

### BACKGROUND OF THE INVENTION

Women experience numerous anatomical, physiological, and hormonal changes during pregnancy that manifest themselves as a variety of symptoms and degrees of discomfort. They may not always report their symptoms to their caregivers as those symptoms may often be normal side effects of a healthy pregnancy. For this reason, expectant mothers are often reluctant continuously to report symptoms if the first symptom they reported was diagnosed by their caregiver as a normal side effect of pregnancy. In particular, they do not wish to be seen as being hypochondriac.

However, as pregnancy progresses, potential risks may develop. Symptoms that may seem harmless could in fact be indicators of a risk condition, depending on contextual factors such as a pattern of different symptoms, pre-conditions of the expectant mother, and/or the gestational age.

For early risk identification, it is important that professional caregivers are alerted as soon as possible. To make this possible, they need immediate reporting of symptoms. By recognizing patterns of symptoms that may signify a potential risk, care providers are able to make early assessment of the potential risk and provide timely medical intervention.

For example, women are told that headaches and vomiting are very common during pregnancy. However, while it is rather common and likely harmless to experience these symptoms in the first trimester, sudden headache and vomiting in the third trimester could be linked to a potential pre-eclampsia condition. If these symptoms appear with blurred vision or with body swelling within a short time, then even if they occur in the first trimester, the presence of these symptoms could be an early warning signal to a serious maternal condition.

According to the World Health Organization, all pregnancies are at risk and around 15% of all pregnant women will develop a potentially life-threatening complications. Timely diagnostic and interventions when signs of risk conditions arise can help to prevent a risk condition to further develop. It is therefore important for women to be aware of and report their symptoms they experience, so that caregivers are able to make earlier diagnostics when combinations of reported symptoms pose an increased level of risk.

WO 2018/071845 discloses a medical data classification system for generating an alert indicating a risk of a pre-term birth. The system particularly aims to identify risks which require external support such as depression and exposure to intimate partner violence.

DE 19955208 discloses a system for monitoring parameters of a pregnant woman. An alarm is generated if measured values exceed stored limits.

There is a need for a system to facilitate this reporting.

### SUMMARY OF THE INVENTION

The invention is defined by the claims.

According to examples in accordance with an aspect of the invention, there is provided a computer-implemented system for alerting an expectant mother to a medical risk during pregnancy, comprising:
a database of pre-stored possible symptoms including at least headache, blurred vision, vomiting, swelling, rashes, joint pain, and fever;
an input interface adapted to receive:
   a profile of the expectant mother, including at least timing information in respect of her pregnancy; and
   reports provided by the expectant mother identifying experienced symptoms from said pre-stored possible symptoms, when those symptoms arise;
a processor; and
an output interface for outputting a risk alert,
wherein the processor is adapted, in response to a report at a particular time, to monitor any reports received over a non-zero time window following that report, and determine, based on the combination of reports received during the time window and the profile of the expectant mother, whether or not to generate and output a risk alert.

There is a problem in ensuring reliable reporting of symptoms for the reasons explained above. The system of the invention encourages reporting of symptoms, because there is no need to involve medical practitioners until a sequence, severity or combination of symptoms is identified by the system as being worthy of a medical report, and for example visit. This is based on analyzing reports in a time window following a first report.

Note that this does not mean the system cannot respond immediately to a symptom which is of sufficient severity to warrant immediate action. Thus, the time window may for example be applied for symptoms other than those which are indicative of a condition which is sufficiently serious that a need for a risk alert is evident immediately.

The processor of the system is programmed to recognize symptoms or patterns of different symptoms to trigger a risk alert. A pre-defined time window is used for assessment, in particular to enable multiple symptoms to be considered in combination. If a risk alert is generated, it is used to advise the expectant mother to contact her antenatal care provider.

The system thus does not provide an actual risk prediction, but rather identifies when conditions are such that a proper medical risk assessment is advisable. This is the purpose of the risk alert.

The invention provides a more reliable indication of potential risk, in particular by encouraging reporting of all symptoms without the need to involve medical practitioners until a potential risk situation is identified.

The user for example has a digital interface to report symptoms they experience or observe. The reported symptoms are aggregated and analyzed by an algorithm within a time window.

The processor is for example adapted to select the time window duration depending on the symptom reported at the particular time.

The time window duration may thus adapt according to the detected symptom. For example, a longer progression of some symptoms such as a fever may need to be analyzed before a risk assessment is made.

The length of the time window is in this example dynamic rather that static. The time window will for example only be opened if a symptom related to a potential underlying risk is reported. It opens the window because the single symptom in itself may not be sufficient to provide a risk alert without further symptom reporting of other symptoms related to the same risk condition.

The length of the time window chosen may thus be based on the optimum window of symptom development considering the potential underlying risk condition.

For analyzing potential patterns or symptoms that may constitute an elevated risk, the algorithm for example considers deviation from the expected physiological development timeline of the corresponding patient group. If a single symptom is reported but does not pose an immediate increased level of risk, the system may for example prompt the user in minutes/hours/days and still within the time window to question user and to make the user aware of checking other related symptom(s) that in combination may potentially link to a risk condition.

The profile of the expectant mother for example further includes one or more of her age, BMI and ethnicity.

These factors enable a risk assessment to take account of the current physiological characteristics of the expectant mother.

The profile of the expectant mother may also include a general risk level associated with her general health or with any specific medical conditions or history for the expectant mother.

This enables a risk assessment to take account of the previous medical history of the expectant mother, for example including information relating to any previous childbirth. It is for example set by a caregiver.

The time window for example has a duration of between 24 hours and 1 week, such as an integer number of days between 1 and 7. The time window duration is for example selected (based on the initial symptom) from a group of durations comprising 1,2,3,4,5,6,7 days. The duration may be selected on the basis of the first self-reported symptom that may be related to an underlying risk. Clinical information related to the particular risk will be used to select an optimal time window. A time window duration of the order of days enables related symptoms (e.g. in the morning and in the evening) to be assessed together.

The reports for example provide an indication of the nature of a symptom and the severity of the symptom, and the risk alert is generated in dependence on the nature, severity, frequency and severity trends of the reported symptoms within the time window.

Of course, if a symptom is extremely severe, a risk alert may be generated immediately. Similarly, a combination of symptoms may trigger a risk alert without needing to wait until the end of the time window.

The processor may be further adapted, in response to a reported symptom, to generate advisory information for output using the output interface presenting advice in respect of alleviating measures or precautionary actions relating to the symptom.

The system thus provides reassurance and information in respect of symptoms that do not trigger a risk alert, and hence this encourages compliance of the expectant mother in reporting the symptoms.

The processor may be further adapted, in response to a reported symptom, to request further input from the expectant mother in respect of related symptoms that she may be experiencing.

In this way, the expectant mother may experience a dominant symptom such as headache but may therefore be unaware (without prompting) of other symptoms such as swelling. By prompting the expectant mother to look for related symptoms the reliability in generating a risk alert is increased.

The system may further comprise a memory for storing all reports and all risk alerts, to create an overall pregnancy profile. This overall pregnancy profile enables detailed analysis of the symptom history, which can be analyzed by a medical practitioner.

The system may further comprise a communications unit for sending any risk alerts automatically to an antenatal care provider of the expectant mother.

In this way, any risk alert can be automatically reported so that a proper medical analysis of the symptom history can then be conducted, even before any physical meeting with the expectant mother. Thus, any urgent medical conditions can be detected more reliably.

The system may comprise a database of pre-stored possible symptoms, from which the expectant mother can select, including at least headache, blurred vision, vomiting, swelling, rashes, joint pain, and fever.

These are symptoms commonly experienced during normal pregnancy, which may be normal side effects but which can also be caused by medical conditions which may arise during pregnancy.

The input interface is for example further adapted to receive physiological parameters of the expectant mother monitored by physiological sensors. In addition to self-reporting, physiological parameters such as heart rate or blood pressure may also be reported automatically.

The system is for example implemented on a mobile phone or tablet. This provides a convenient user interface for the expectant mother.

The invention also provides a processor for generating a risk alert for alerting an expectant mother to a medical risk during pregnancy, comprising:
an input adapted to receive profile data of the expectant mother, including at least timing information in respect of her pregnancy and reports provided by the expectant mother identifying experienced symptoms, wherein said experienced symptoms are selected from a database of pre-stored possible symptoms including at least headache, blurred vision, vomiting, swelling, rashes, joint pain, and fever,
wherein the processor is adapted, in response to a report at a particular time, to monitor any reports received over a non-zero time window following that report, and determine, based on the combination of reports received during the time window and the profile of the expectant mother, whether or not to generate a risk alert.

This processor may be located at a user terminal or it may be remote from the user terminal.

The invention also provides a computer-implemented method for alerting an expectant mother to a medical risk, comprising:
providing a profile of the expectant mother to a computer-implemented system, including at least timing information in respect of her pregnancy;
providing reports to the computer-implemented system identifying symptoms experienced by the expectant mother, wherein said experienced symptoms are selected from a database of pre-stored possible symptoms including at least headache, blurred vision, vomiting, swelling, rashes, joint pain, and fever;
using a processor of the computer-implemented system, in response to a report at a particular time, to monitor any reports received over a non-zero time window following that report, and determine, based on the combination of reports received during the time window and the profile of the expectant mother, whether or not to generate and output a risk alert; and
outputting any generated alert to the expectant mother.

Providing a profile of the expectant mother for example comprises providing one or more of her age, BMI and ethnicity, and a general risk level associated with the general health or specific medical conditions or history for that expectant mother.

Providing reports for example comprises providing an indication of the nature of a symptom and the severity of the symptom, and the risk alert is generated in dependence on the nature, severity, frequency and trends of the reported symptom within the time window.

The invention also provides a computer program comprising computer program code means which is adapted, when said program is run on a computer, to carry out the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a computer-implemented system for alerting an expectant mother to a medical risk during pregnancy; and
Figure 2 shows a computer-implemented method for alerting an expectant mother to a medical risk during pregnancy.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented system and method for alerting an expectant mother to a medical risk during pregnancy. A profile of the expectant mother is used, and reports are received from the expectant mother identifying experienced symptoms. In response to a report at a particular time, any reports received over a subsequent time window are monitored, and based on the combination of reports received during the time window and the profile of the expectant mother, the need for a risk alert is determined. The user is more willing to report symptoms, because a risk alert (which functions as reporting symptoms to a medical expert) only takes place when a real risk is identified.

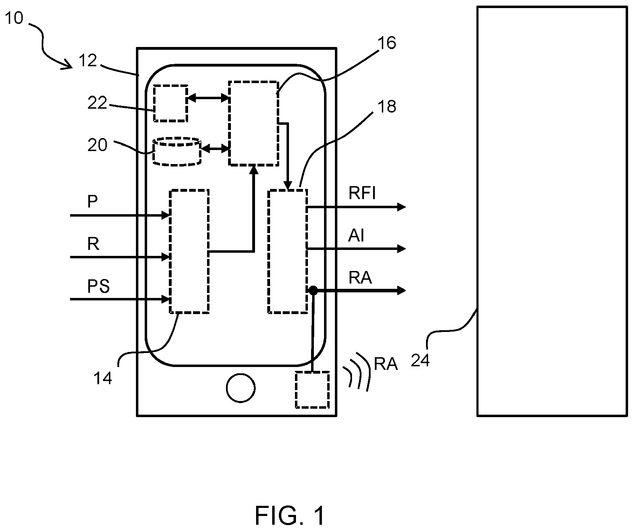

Figure 1 shows a computer-implemented system 10 for alerting an expectant mother to a medical risk during pregnancy. The system comprises a user terminal 12, which is for example in the form of a smartphone or tablet on which a suitable computer program is loaded.

The user terminal comprises an input interface 14 which is adapted to receive inputs from the user. These inputs may be input manually or they may be received automatically from sensors or other sources of information.

The inputs comprise a profile, P, of the expectant mother, including at least timing information in respect of her pregnancy, i.e. the gestational stage of her pregnancy. This profile typically also includes her age, BMI and ethnicity. The profile may also include a general risk level associated with her general health or with any specific medical conditions or history.

The inputs also include reports, R, provided by the expectant mother identifying experienced symptoms. These reports may be provided manually by the expectant mother, but to simplify use of the system, they may be selected from a menu of possible symptoms supported by the system. For this purpose, there is database 20 of pre-stored possible symptoms, from which the expectant mother can select. This database is in practice simply an area of memory of the mobile phone or tablet in which data is stored. The pre-stored possible symptoms for example include at least headache, blurred vision, vomiting, swelling, rashes, joint pain, fever and bleeding.

The reports may not only identify the nature of a symptom but also indicate the severity of the symptom, so that a progression of symptom severity can be tracked.

The input interface 14 further optionally receives physiological sensing parameters, PS, of the expectant mother monitored by physiological sensors. These may for example comprise blood pressure and heart rate.

The input interface is typically the touch screen or a separate keyboard of the device, although any suitable input interface may be used such as using voice recognition.

The user terminal has a processor 16 and an output interface 18, most importantly for outputting a risk alert, RA.

The output information may additionally comprise advisory information, AI, presenting advice in respect of alleviating measures or precautionary actions relating to the symptom. The output information may further comprise a request for further input, RFI, from the expectant mother in respect of related symptoms that she may be experiencing. In this way, the system can check if there are multiple related symptoms.

The user terminal also comprises a memory 22 for storing all reports (and all risk alerts, discussed below) to create an overall pregnancy profile as a timeline of events. This may be used by a medical practitioner to provide a full history of the pregnancy.

The processor is the processor of the mobile phone or tablet and the output interface is the screen and microphone, or else the output interface may comprise a wireless communications system for communicating with another remote device.

In order to determine if a risk warning is needed, the processor 16 starts a time window when a first report of a symptom is received, e.g. following a period of no reports or after the end of any previous time window. The time window may have fixed duration, or it may have a duration which depends on the nature of the first symptom recorded. During this time window, any reports received following that first report are monitored. Based on the combination of reports (and the optional physiological parameter sensing) received during the time window, and the profile of the expectant mother, it is determined whether or not to generate and output a risk alert. A risk alert is generated in dependence on the nature, severity, frequency and trends of the reported symptoms and physiological parameters within the time window.

If a symptom reported is severe by itself, a risk alert can be generated immediately. However, if the symptom is not sufficiently severe, because it may be a normal side effect to be expected (based on the other profile information), then the monitoring continues for the remainder of the time window. If the progression of symptoms or physiological parameters indicates that in fact there is a risk of a medical complication, the risk alert is generated.

The time window for example has a duration of between 1 day and 1 week, such as between 2 days and 4 days, such as 3 days.

Figure 1 also shows a computer system 24 of an antenatal care provider. Any risk alerts RA are also automatically sent to the antenatal care provider of the expectant mother, i.e. to the computer system 24.

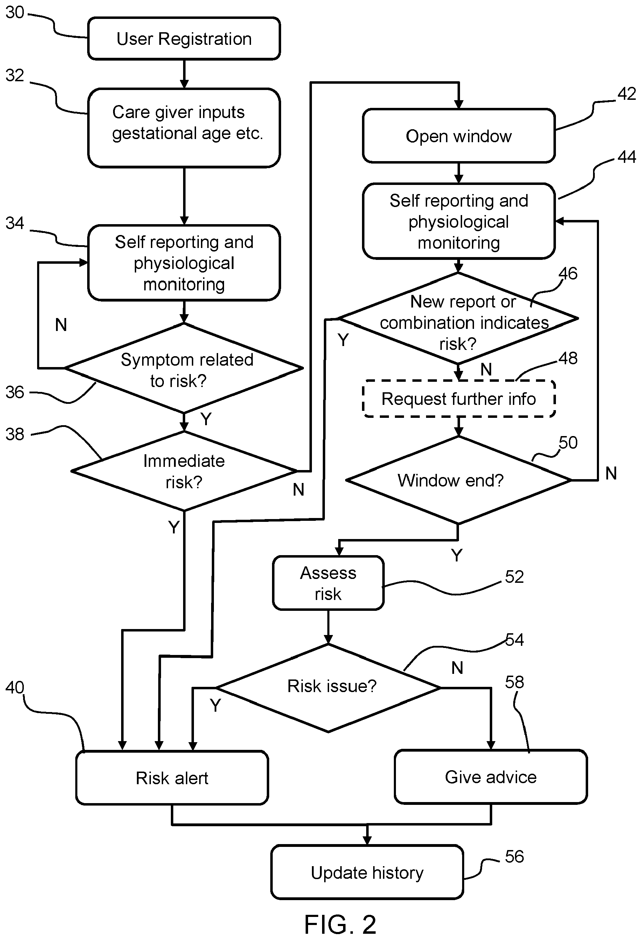

Figure 2 shows a method for alerting an expectant mother to a medical risk.

In step 30, the expectant mother registers with the system. This involves inputting part of the data that forms the user profile. A health care professional for example introduces the system to the expectant mother and instructs her to register, by filling in her personal information via the app running on the user terminal.

In step 32, the caregiver receives the registration notice via a professional carer app interface on the system 24. The registration notice confirms that a client has registered in the system.

The health care professional then enters the gestational age (after a scan) and any remaining information for the user profile, such as other related medical information. This activates the reporting cycle.

Thus, a profile of the expectant mother has then been input to the system, including at least timing information in respect of the pregnancy, for example age, expected due date, BMI, ethnicity/race, language preference etc. It may include existing medical conditions and history.

In step 32, a risk profile may also be selected for that user, e.g. high risk, low risk or medium risk. This may depend on the medical history of the user and any finding from blood tests or ultrasound imaging of the fetus. The risk level for example sets the thresholds at which a risk alert is triggered in a user-dependent manner. The risk level is for example selected by the caregiver.

The description that follows applies to all risk profiles, but the determination of whether a risk alert is considered appropriate will take account of that risk profile.

In step 34 the user (expectant mother) is able to provide a report to the system identifying symptoms experienced. This may be based on a drop down menu system, for example. The method waits for a symptom to be reported in step 36, although automatic physiological monitoring may indicate that a symptom is present. In step 36, it is determined if the symptom that has been reported relates to a potential risk.

If the symptom is related to a potential risk, it is determined in step 38 whether the reported symptom is sufficiently serious for a risk alert to generated straight away, in step 40.

If the symptom is not indicative of an emergency and hence immediate reporting is not needed, a time window is opened, in step 42. In step 44, the user is again able to provide further reports to the system identifying symptoms experienced. For any reported symptom, it is determined in step 46 if, in combination with the previously reported symptoms within the time window, a risk alert should be made. Alternatively, if the newly reported symptom is sufficiently serious alone, a risk alert is needed, (e.g. a bleed), In either case, the method can jump to step 40 when a risk alert is needed.

If not, there is continued physiological monitoring during the time window, if physiological monitoring is used. During the time period of the time window, the user may optionally be requested to consider if other specific symptoms are present, in step 48, to assist the system in gathering all relevant information. This may for example be particularly relevant if some symptoms are repeatedly being reported or with increasing severity. Even if symptoms are all mild, they may in combination be indicative of a more serious condition.

This continues, until the time window has ended as determined in step 50.

Once a time window expires, a new one will be opened by a new reporting of a subsequent symptom by the user. Thus, once the time window has expired without a risk alert, a new time window can start when there is a next reported symptom. In the example described herein, the algorithm will not consider reports that occurred in a previous time window in order to make the risk assessment. However, all reporting will be logged, and if a risk flag is eventually raised (in a new cycle), health care professionals can review the onset of previous reported symptoms to assess the potential risk level.

The purpose of the system is basically to flag an imminent emerging risk before it becomes an acute condition, and typically before a next scheduled medical meeting.

Note that a time window of around 3 days is considered suitable for most risk conditions (except a small number of risks that may require a window of different length).

Risks that develop more slowly (i.e. over a longer period of time than the time window) will be identified during regularly scheduled (e.g. weekly or fortnightly) antenatal care visits. For example, the World Health Organization's antenatal care model recommends that all pregnant women should have eight contacts with a health provider throughout pregnancy.

The time window may instead be thought of as a moving window, so that for any reported symptom, the immediately preceding history for the already-existing duration of the time window is take into account in determining whether there is a need for a risk alert. Thus, the analysis looks back at previous history.

Once a symptom related to a potential risk has been used to open the time window, the prompting of the user interface will try to pro-actively establish if a user may be experiencing any other symptoms related to the same underlying risk.

For the example represented in Figure 2, at the end of the time window, a (further) risk assessment is made in step 52. This is of course optional, if all required risk assessment has been completed in step 46. This may take account of the risk profile of the user.

Each risk assessment (e.g. in step 46 or in the final assessment in step 52) takes account of the nature of the reported (or detected) symptoms and the severity of the symptoms, as well as the frequency and trends of the symptoms within the time window. The risk assessment involves identifying deviations from the expected physiological development timeline of the patient group corresponding to the user, such as the patient group for that particular gestational age etc. Thus, there is mapping of reported symptoms to the expected physiological development of the user, and the expected development of symptoms of the user associated with the progression of a pregnancy. The invention provides real time analysis for enabling early intervention.

In step 54, it is then determined if there is an issue to report. This may for example be based on an increased overall risk level. If a risk alert is needed, it is reported in step 40. This report may be to the user and to a caregiver of the user. The user history is then updated in step 56.

If in step 54 it is determined that there is no need to issue a risk alert, other actions may still be taken. In step 58, output information may be provided to the user, for example giving tips or advice about avoiding or alleviating the symptoms. For example, tips may be given about dealing with nausea. Advice may be given about medication that should or should not be taken e.g. as a headache remedy. Note that this step 58 may also take place during the time window (e.g. with step 48), not only at the end as shown.

The user history is then updated in step 56. Thus, regardless of whether a risk alert is needed, all information is stored as part of the user history.

Thus, the operation of the system overall is that in response to a report, any reports received over a time window following that report are monitored, and based on the combination of reports received during the time window and the profile of the expectant mother, it is determined whether or not to generate and output a risk alert. The algorithm in particular recognizes symptoms or patterns of different symptoms, such as identified in national health guidelines, in order to trigger an alert.

When the user (pregnant woman) reports symptoms the algorithm checks whether the reported symptom may be linked to a potential risk. If it finds a positive correlation, the system prompts the user (in step 48) and asks questions to determined whether she has other related symptoms that in combination would increase the chance of that risk condition. The questions are provided with the pre-defined time window after a symptom is actively reported by the woman. This takes account of the fact that not all symptoms of a risk condition often appear at once.

By way of example, a pregnant woman in her 20th week of pregnancy may vomit in the morning but only have severe headache and blurred vision starting later in the day. If the user experiences vomiting in the morning but does not have other symptoms, she will naturally only report vomiting as the only symptom of her condition. When she experiences headache and blurred vision later on during the day, she may not naturally feel the need to report as she may associate that as further side effects linked to the first symptoms.

The time window allows the system to prompt the user to check whether her last reported symptom still persists and whether she has additional signs of symptoms that could be linked to a risk condition. The prompting method enables pregnant women to feel at ease in reporting symptoms without being stigmatized as being paranoid. It also helps the user to be aware of their symptoms.

As antenatal care visits are typically scheduled over weekly intervals, another advantage of the invention is to be able to alert both user and caregiver for diagnostics, that are earlier than scheduled visits. This system also helps to keep track of all symptoms reported by the woman throughout her pregnancy, so that professional caregivers can use this additional information to create a holistic view of the patient case.

It is noted that the processing carried out by the system may be performed within the user terminal or it may be performed remotely. For example, the processor may be on a remote server and the phone or tablet is essentially only used as interface to the server. The processing may take place at one location or it may be distributed. The system is of particular interest when health care professionals (midwife / obstetrician) monitor any risk alerts generated by the user terminal. Thus, the health care professional uses their own a care provider interface to access the system, and the care provider is also registered to the system. Thus, the expectant mother women is registered with a health care professional who will monitor her symptoms as reported via the system.

The input interface and output interface have been discussed above. In each case, the "interface" may support different types of input and output. For example, data may be input manually by interaction with the device or by data communication from a remote device. There may be multiple modes of inputting data, such as by sound, touch or by connection to a keyboard. Similarly, data may be output directly from the device (by a screen and/or speaker) or by sending data to another output device.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. The computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented system (10) for alerting an expectant mother to a medical risk during pregnancy, comprising:
a database (20) of pre-stored possible symptoms including at least headache, blurred vision, vomiting, swelling, rashes, joint pain, and fever;
an input interface (14) adapted to receive:
a profile (P) of the expectant mother, including at least timing information in respect of her pregnancy; and
reports (R) provided by the expectant mother identifying experienced symptoms from said pre-stored possible symptoms, when those symptoms arise;
a processor (16); and
an output interface (18) for outputting a risk alert (RA),
wherein the processor is adapted, in response to a report at a particular time, to monitor any reports received over a non-zero time window following that report, and determine, based on the combination of reports received during the time window and the profile of the expectant mother, whether or not to generate and output a risk alert.

2. A system as claimed in claim 1, wherein the processor (16) is adapted to select the time window duration depending on the symptom reported at the particular time.

3. A system as claimed in claim 1 or 2, wherein the profile (P) of the expectant mother further includes one or more of her age, BMI and ethnicity.

4. A system as claimed in any one of claims 1 to 3, wherein the profile (P) of the expectant mother includes a general risk level associated with her general health or with any specific medical conditions or history for the expectant mother.

5. A system as claimed in any one of claims 1 to 4, wherein the time window has a duration of between 24 hours and 1 week, such as an integer number of days from 1 to 7.

6. A system as claimed in any one of claims 1 to 5, wherein the reports (R) provide an indication of the nature of a symptom and the severity of the symptom, and the risk alert is generated in dependence on the nature, severity, frequency and trends of the reported symptoms within the time window.

7. A system as claimed in any one of claims 1 to 6, wherein the processor (16) is further adapted, in response to a reported symptom:
to generate advisory information for output using the output interface presenting advice in respect of alleviating measures or precautionary actions relating to the symptom; and/or
to request further input from the expectant mother in respect of related symptoms that she may be experiencing.

8. A system as claimed in any one of claims 1 to 7, further comprising a memory (22) for storing all reports and all risk alerts, to create an overall pregnancy profile.

9. A system as claimed in any one of claims 1 to 8, further comprising a communications unit for sending any risk alerts automatically to an antenatal care provider of the expectant mother.

10. A system as claimed in any one of claims 1 to 9, wherein the input interface (14) is further adapted to receive physiological parameters of the expectant mother monitored by physiological sensors.

11. A system as claimed in any one of claims 1 to 10, wherein the input interface and the output interface are implemented on a mobile phone or tablet.

12. A processor for generating a risk alert for alerting an expectant mother to a medical risk during pregnancy, comprising:
an input adapted to receive profile data of the expectant mother, including at least timing information in respect of her pregnancy and reports provided by the expectant mother identifying experienced symptoms, wherein said experienced symptoms are selected from a database (20) of pre-stored possible symptoms including at least headache, blurred vision, vomiting, swelling, rashes, joint pain, and fever,
wherein the processor is adapted, in response to a report at a particular time, to monitor any reports received over a non-zero time window following that report, and determine, based on the combination of reports received during the time window and the profile of the expectant mother, whether or not to generate a risk alert.

13. A computer-implemented method for alerting an expectant mother to a medical risk, comprising:
(30) providing a profile of the expectant mother to a computer-implemented system, including at least timing information in respect of her pregnancy;
(34, 44) providing reports to the computer-implemented system identifying symptoms experienced by the expectant mother, wherein said experienced symptoms are selected from a database (20) of pre-stored possible symptoms including at least headache, blurred vision, vomiting, swelling, rashes, joint pain, and fever;
using a processor of the computer-implemented system, in response to a report at a particular time, to monitor any reports received over a non-zero time window following that report, and determine, based on the combination of reports received during the time window and the profile of the expectant mother, whether or not to generate and output a risk alert; and
(40) outputting any generated alert to the expectant mother.

14. A computer program comprising computer program code means which is adapted, when said program is run on a computer, to carry out the method of claim 13.

## Patentansprüche

1. Computerimplementiertes System (10) zum Warnen einer werdenden Mutter vor einem medizinischen Risiko während einer Schwangerschaft, umfassend:
eine Datenbank (20) mit vorab gespeicherten möglichen Symptomen, die mindestens Kopfschmerzen, verschwommenes Sehen, Erbrechen, Schwellungen, Hautausschläge, Gelenkschmerzen und Fieber beinhaltet;
eine Eingangsschnittstelle (14), die angepasst ist, um Folgendes zu empfangen:
ein Profil (P) der werdenden Mutter, das zumindest zeitliche Informationen in Bezug auf ihre Schwangerschaft beinhaltet, und
Berichte (R), die von der werdenden Mutter bereitgestellt werden, die erlebte Symptome aus den zuvor gespeicherten möglichen Symptomen identifizieren, wenn diese Symptome auftreten;
einen Prozessor (16); und
eine Ausgabeschnittstelle (18) zum Ausgeben einer Risikowarnung (RA),
wobei der Prozessor angepasst ist, um als Reaktion auf einen Bericht zu einem bestimmten Zeitpunkt alle Berichte zu überwachen, die über ein Zeitfenster ungleich null nach diesem Bericht empfangen werden, und basierend auf der Kombination der während des Zeitfensters empfangenen Berichte und des Profils der werdenden Mutter zu bestimmen, ob eine Risikowarnung erzeugt und ausgegeben werden soll.

2. System nach Anspruch 1, wobei der Prozessor (16) angepasst ist, um die Dauer des Zeitfensters abhängig von dem zu einem bestimmten Zeitpunkt berichteten Symptom auszuwählen.

3. System nach Anspruch 1 oder 2, wobei das Profil (P) der werdenden Mutter ferner eines oder mehrere von ihrem Alter, BMI und ethnische Zugehörigkeit beinhaltet.

4. System nach einem der Ansprüche 1 bis 3, wobei das Profil (P) der werdenden Mutter ein allgemeines Risikoniveau beinhaltet, das mit ihrem allgemeinen Gesundheitszustand oder mit spezifischen medizinischen Bedingungen oder der Vorgeschichte der werdenden Mutter assoziiert ist.

5. System nach einem der Ansprüche 1 bis 4, wobei das Zeitfenster eine Dauer zwischen 24 Stunden und einer Woche aufweist, wie z.B. eine ganzzahlige Anzahl von Tagen von 1 bis 7.

6. System nach einem der Ansprüche 1 bis 5, wobei die Berichte (R) einen Hinweis auf die Art eines Symptoms und den Schweregrad des Symptoms bereitstellen und die Risikowarnung abhängig von der Art, dem Schweregrad, der Häufigkeit und den Trends der berichteten Symptome innerhalb des Zeitfensters erzeugt wird.

7. System nach einem der Ansprüche 1 bis 6, wobei der Prozessor (16) als Reaktion auf ein berichtetes Symptom ferner zu Folgendem angepasst ist:
Erzeugen von Beratungsinformationen zur Ausgabe unter Verwendung der Ausgabeschnittstelle, die Ratschläge in Bezug auf lindernde Maßnahmen oder Vorsichtsmaßnahmen in Bezug auf das Symptom enthalten; und/oder
Auffordern der werdenden Mutter, weitere Angaben zu den Symptomen zu machen, unter denen sie möglicherweise leidet.

8. System nach einem der Ansprüche 1 bis 7, ferner umfassend einen Speicher (22) zum Speichern aller Berichte und aller Risikowarnungen, um ein umfassendes Schwangerschaftsprofil zu erstellen.

9. System nach einem der Ansprüche 1 bis 8, ferner umfassend eine Kommunikationseinheit zum automatischen Senden von Risikowarnungen an einen Anbieter von Schwangerschaftsbetreuung für die werdende Mutter.

10. System nach einem der Ansprüche 1 bis 9, wobei die Eingabeschnittstelle (14) ferner angepasst ist, um physiologische Parameter der werdenden Mutter zu empfangen, die von physiologischen Sensoren überwacht werden.

11. System nach einem der Ansprüche 1 bis 10, wobei die Eingabeschnittstelle und die Ausgabeschnittstelle auf einem Mobiltelefon oder Tablet implementiert sind.

12. Prozessor zum Erzeugen einer Risikowarnung, um eine werdende Mutter vor einem medizinischen Risiko während einer Schwangerschaft zu warnen, umfassend:
einen Eingang, der angepasst ist, um Profildaten der werdenden Mutter zu empfangen, einschließlich zumindest zeitlicher Informationen in Bezug auf ihre Schwangerschaft und von der werdenden Mutter bereitgestellter Berichte, die erlebte Symptome identifizieren, wobei die erlebten Symptome ausgewählt sind aus einer Datenbank (20) vorgespeicherter möglicher Symptome, die zumindest Kopfschmerzen, verschwommenes Sehen, Erbrechen, Schwellungen, Ausschläge, Gelenkschmerzen und Fieber beinhalten,
wobei der Prozessor angepasst ist, um als Reaktion auf einen Bericht zu einem bestimmten Zeitpunkt alle Berichte zu überwachen, die über ein Zeitfenster ungleich null nach diesem Bericht empfangen werden, und basierend auf der Kombination der während des Zeitfensters empfangenen Berichte und des Profils der werdenden Mutter zu bestimmen, ob eine Risikowarnung erzeugt werden soll.

13. Computerimplementiertes Verfahren zum Warnen einer werdenden Mutter vor einem medizinischen Risiko, umfassend:
(30) Bereitstellen eines Profils der werdenden Mutter an ein computerimplementiertes System, das zumindest zeitliche Informationen in Bezug auf ihre Schwangerschaft beinhaltet,
(34, 44) Bereitstellen von Berichten an das computerimplementierte System, die von der werdenden Mutter erlebte Symptome identifizieren, wobei die erlebten Symptome ausgewählt sind aus einer Datenbank (20) vorgespeicherter möglicher Symptome, die zumindest Kopfschmerzen, verschwommenes Sehen, Erbrechen, Schwellungen, Hautausschläge, Gelenkschmerzen und Fieber beinhalten;
Verwenden eines Prozessors des computerimplementierten Systems, um als Reaktion auf einen Bericht zu einem bestimmten Zeitpunkt alle Berichte zu überwachen, die über ein Zeitfenster ungleich null nach diesem Bericht empfangen werden, und basierend auf der Kombination der während des Zeitfensters empfangenen Berichte und des Profils der werdenden Mutter zu bestimmen, ob eine Risikowarnung erzeugt und ausgegeben werden soll; und
(40) Ausgeben einer erzeugten Warnung an die werdende Mutter.

14. Computerprogramm, umfassend Computerprogrammcode-Einrichtungen, die angepasst sind, um, wenn das Programm auf einem Computer ausgeführt wird, das Verfahren nach Anspruch 13 durchzuführen.

## Revendications

1. Système informatique (10) pour alerter une femme enceinte d'un risque médical pendant la grossesse, comprenant:
une base de données (20) de symptômes possibles préenregistrés comprenant au moins des maux de tête, une vision floue, des vomissements, un gonflement, des éruptions cutanées, des douleurs articulaires et de la fièvre;
une interface d'entrée (14) adaptée pour recevoir:
un profil (P) de la femme enceinte, comprenant au moins des informations temporelles concernant sa grossesse; et des rapports (R) fournis par la femme enceinte identifiant les symptômes éprouvés à partir desdits symptômes possibles préenregistrés, lorsque ces symptômes apparaissent;
un processeur (16); et
une interface de sortie (18) pour sortir une alerte de risque (RA), dans lequel le processeur est adapté, en réponse à un rapport à un moment particulier, pour surveiller tous les rapports reçus sur une fenêtre de temps non nulle suivant ce rapport, et déterminer, sur la base de la combinaison des rapports reçus pendant la fenêtre de temps et du profil de la femme enceinte, s'il faut ou non générer et émettre une alerte de risque.

2. Système selon la revendication 1, dans lequel le processeur (16) est adapté pour sélectionner la durée de la fenêtre temporelle en fonction du symptôme rapporté à l'instant particulier.

3. Système selon la revendication 1 ou 2, dans lequel le profil (P) de la femme enceinte comprend en outre un ou plusieurs parmi son âge, son BMI et son origine ethnique.

4. Système selon l'une quelconque des revendications 1 à 3, dans lequel le profil (P) de la femme enceinte comprend un niveau de risque général associé à son état de santé général ou à toute condition médicale ou historique spécifique de la femme enceinte.

5. Système selon l'une quelconque des revendications 1 à 4, dans lequel la fenêtre temporelle a une durée comprise entre 24 heures et 1 semaine, tel qu'un nombre entier de jours de 1 à 7.

6. Système selon l'une quelconque des revendications 1 à 5, dans lequel les rapports (R) fournissent une indication de la nature d'un symptôme et de la gravité du symptôme, et l'alerte de risque est générée en fonction de la nature, de la gravité, la fréquence et les tendances des symptômes signalés dans la fenêtre temporelle.

7. Système selon l'une quelconque des revendications 1 à 6, dans lequel le processeur (16) est en outre adapté, en réponse à un symptôme signalé:
pour générer des informations de conseil pour la sortie à l'aide de l'interface de sortie présentant des conseils concernant des mesures d'atténuation ou des actions de précaution relatives au symptôme; et/ou
pour demander des informations supplémentaires à la femme enceinte en ce qui concerne les symptômes connexes qu'elle peut ressentir.

8. Système selon l'une quelconque des revendications 1 à 7, comprenant en outre une mémoire (22) pour stocker tous les rapports et toutes les alertes de risque, pour créer un profil de grossesse global.

9. Système selon l'une quelconque des revendications 1 à 8, comprenant en outre une unité de communication pour envoyer automatiquement toute alerte de risque à un prestataire de soins prénatals de la femme enceinte.

10. Système selon l'une quelconque des revendications 1 à 9, dans lequel l'interface d'entrée (14) est en outre adaptée pour recevoir des paramètres physiologiques de la femme enceinte surveillés par des capteurs physiologiques.

11. Système selon l'une quelconque des revendications 1 à 10, dans lequel l'interface d'entrée et l'interface de sortie sont mises en oeuvre sur un téléphone mobile ou une tablette.

12. Processeur pour générer une alerte de risque pour alerter une femme enceinte d'un risque médical pendant la grossesse, comprenant:
une entrée adaptée pour recevoir des données de profil de la femme enceinte, comprenant au moins des informations temporelles concernant sa grossesse et des rapports fournis par la femme enceinte identifiant les symptômes ressentis, dans lequel lesdits symptômes ressentis sont sélectionnés à partir d'une base de données (20) de données possibles préenregistrées symptômes comprenant au moins des maux de tête, une vision floue, des vomissements, un gonflement, des éruptions cutanées, des douleurs articulaires et de la fièvre,
dans lequel le processeur est adapté, en réponse à un rapport à un moment particulier, pour surveiller tous les rapports reçus sur une fenêtre de temps non nulle suivant ce rapport, et déterminer, sur la base de la combinaison des rapports reçus pendant la fenêtre de temps et du profil de la femme enceinte, de générer ou non une alerte de risque.

13. Procédé mis en oeuvre par ordinateur pour alerter une femme enceinte d'un risque médical, comprenant:
(30) fournir un profil de la femme enceinte à un système mis en oeuvre par ordinateur, comprenant au moins des informations temporelles concernant sa grossesse;
(34, 44) fournissant des rapports au système mis en oeuvre par ordinateur identifiant les symptômes ressentis par la femme enceinte, dans lequel lesdits symptômes ressentis sont sélectionnés à partir d'une base de données (20) de symptômes possibles préenregistrés comprenant au moins des maux de tête, une vision floue, des vomissements, un gonflement, éruptions cutanées, douleurs articulaires et fièvre;
l'utilisation d'un processeur du système mis en oeuvre par ordinateur, en réponse à un rapport à un moment particulier, pour surveiller tous les rapports reçus sur une fenêtre de temps non nulle suivant ce rapport, et déterminer, sur la base de la combinaison des rapports reçus pendant la fenêtre de temps et le profil de la femme enceinte, s'il faut ou non générer et émettre une alerte de risque; et
(40) délivrer toute alerte générée à la femme enceinte.

14. Programme d'ordinateur comprenant des moyens de code de programme d'ordinateur qui est adapté, lorsque ledit programme est exécuté sur un ordinateur, à mettre en oeuvre le procédé de la revendication 13.
